# EUROPEAN PATENT APPLICATION

(11) **EP 4 321 160 A1**
(43) Date of publication of application: **14.02.2024**
(21) Application number: 22784859.5
(22) Date of filing: 31.03.2022
(51) Int. Cl.: A61K 31/4375, A61K 31/352, A61P 3/10, A23L 33/10

(54) **COMPOUND FOR PREVENTING OR TREATING DIABETES**

(30) Priority: 06.04.2021 KR 20210044582
(71) Applicant: Oncocross Co., Ltd., Seoul 04168 (KR)
(72) Inventor: KIM, Yi Rang, Sejong 30064 (KR)
(74) Representative: Isarpatent
(86) International application number: PCT/KR2022/004614
(87) International publication number: WO 2022/215949

(57) **Abstract**

The present invention relates to a use of (R)-4-{(R)-1-[7-(3,4,5-trimethoxyphenyl)-[1,6] naphthyridine-5-yloxy]-ethyl}pyrrolidine-2-one for treating diabetes. According to the present invention, (R)-4-{(R)-1-[7-(3,4,5-trimethoxy-phenyl)-[1,6] naphthyridine-5-yloxy]-ethyl}pyrrolidine-2-one of the present invention alleviates clinical symptoms of diabetes, reduces blood sugar, reduces blood glucose concentrations and insulin-resistance and reduces liver and kidney damages in an animal model of insulin-resistant diabetes, and thus can be effectively used for preventing or treating diabetes, especially insulin-resistant diabetes.

## Description

### [Technical Field]

The present disclosure relates to a use of (R)-4-{(R)-1-[7-(3,4,5-trimethoxyphenyl)-[1,6] naphthyridine-5-yloxy]-ethyl}pyrrolidine-2-one for treating diabetes.

### [Background Art]

Diabetes mellitus is a metabolic disease characterized by hyperglycemia caused by defects in insulin secretion or insulin action, and a chronic degenerative disease that occurs due to a lack of insulin or a decrease in a biological effect of insulin in target cells and persists for a long time, resulting in complications such as renal failure. The diabetes mellitus causes various complications from the early stages, and is accompanied by acute complications such as hypoglycemia, ketoacidosis, diabetic coma, local and systemic bacterial infection, and chronic complications such as diabetic nephropathy, hypertension, diabetic retinopathy, cataracts, neuropathy, and cardiovascular complications. The diabetes mellitus is the third most serious disease worldwide and the fourth leading cause of death in Korea, and shortens the lifespan by 5 to 10 years due to various complications. The diabetes mellitus is a metabolic disorder syndrome characterized by hyperglycemia caused by defects in insulin action, insulin secretion, or both, and as hyperglycemia continues chronically, the hyperglycemia causes disorders in not only carbohydrate metabolism but also lipid and protein metabolisms so that complications in the retina, kidneys, nerves, cardiovascular system, etc., are caused resulting in fatal problems such as pain and shortened lifespan for patients. Recently, the number of patients with diabetes mellitus is increasing, while the importance of developing therapeutic agents for diabetes mellitus is emphasized in situations where continuous and appropriate treatment is difficult. The diabetes includes Type 1 diabetes in which pancreatic beta cells are destroyed and insulin secretion is absolutely lacking, and Type 2 diabetes in which although insulin from the pancreas is sufficient or even more than normal, the action thereof is not smooth so that blood sugar (called insulin resistance) is increased or a smaller amount of insulin than an amount required in the pancreas is not secreted so that blood sugar is increased. The Type 2 diabetes is caused by insulin resistance, and for reason, there are no significant symptoms in the early stages by increasing insulin secretion from the pancreas, but when the capacity of the pancreas becomes limited, the symptoms appear and diagnosis is delayed.

The insulin resistance is a condition in which the action of insulin in peripheral tissues is reduced and is the main cause of Type 2 diabetes (Kahn, S.E., Diabetologia, 46, pp3-19, 2003). The diabetes exhibits characteristic symptoms of hyperglycemia are displayed due to abnormal physiological metabolic control functions, such as carbohydrate, protein, lipid and electrolyte metabolisms due to hormonal imbalance including insulin (Gonuth, S.M., Ann. Intern. Med. , 79, pp812-822, 1973). When these hyperglycemia symptoms persist, the symptoms cause blood circulation disorders, retinal damage, nerve cell damage, decreased kidney function, and vascular complications (UK Prospective Diabetes Study (UKPDS) Group, Lancet, 352, pp837853, 1998). Compared to Type 1 diabetes, most patients with Type 2 diabetes do not have severe symptoms and are less likely to develop a complication called ketoacidosis. In many cases, Type 2 diabetes may be treated with diet therapy, exercise therapy, and hypoglycemic agent, but in some cases, blood sugar may also be controlled only with insulin injections.

Currently, when a drug treatment method of Type 2 diabetes, reevaluated metformin and thiazolidinedione (TZD)-based drugs show considerable usefulness, but treatment of the fundamental causes of diabetes, such as the occurrence of insulin resistance, has not been achieved, and various side effects have also been reported, so that there is a need to develop more effective and safer drugs capable of fundamentally solving insulin resistance. The ultimate goal of current diabetes treatment is to continuously maintain normalization of blood sugar. This can be seen from several experimental examples that most diabetic complications are caused by metabolic failure caused by persistent hyperglycemia, and complications may be suppressed or delayed by strict blood sugar control in animals and clinical practice. Diabetes therapeutic agents used clinically are broadly divided into 1) drugs that promote insulin secretion, 2) drugs that enhance the sensitivity of insulin receptors, and 3) drugs that inhibit absorption by inhibiting glycolysis. Insulin preparations have the same physiological effects as in vivo insulin, but have the inconvenience to be administered by injection, and a problem in insulin resistance. Sulfonylurea-based drugs (glibenclamide, glipizide, gliquidone, etc.) are oral and inexpensive, but have problems such as induction of hypoglycemia, and loss of insulin secretion ability. Biguanide-based drugs (metformin, phenformin, etc.) are problematic for gastrointestinal disorders and nephrotoxicity, and glitazone-based drugs (troglitazone, pioglitazone, rosiglitazone, etc.) are appealed from side effects such as heart failure and anemia or liver failure or the sales are suspended due to liver failure. Accordingly, there is an urgent need to develop drugs capable of ensuring both safety and efficacy.

### [Disclosure]

### [Technical Problem]

An object of the present disclosure is to provide a pharmaceutical composition for preventing or treating diabetes.

Another object of the present disclosure is to provide a pharmaceutical composition for preventing or treating diabetic complications.

Yet another object of the present disclosure is to provide a pharmaceutical composition for preventing or treating diabetes-related metabolic abnormalities.

Yet another object of the present disclosure is to provide a health functional food for preventing or alleviating diabetes.

Yet another object of the present disclosure is to provide a method for treating diabetes.

Yet another object of the present disclosure is to provide a use for use in the preparation of a pharmaceutical composition for preventing or treating diabetes.

### [Technical Solution]

One aspect of the present disclosure provides a pharmaceutical composition for preventing or treating diabetes containing (R)-4-{(R)-1-[7-(3,4,5-trimethoxyphenyl)-[1,6] naphthyridine-5-yloxy]-ethyl}pyrrolidine-2-one or a pharmaceutically acceptable salt thereof as an active ingredient.

Another aspect of the present disclosure provides a pharmaceutical composition for preventing or treating diabetic complications containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

Yet another aspect of the present disclosure provides a pharmaceutical composition for preventing or treating diabetes-related metabolic abnormalities containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

Yet another aspect of the present disclosure provides a health functional food for preventing or alleviating diabetes containing (R)-4-{(R)-1-[7-(3,4,5-trimethoxyphenyl)-[1,6] naphthyridine-5-yloxy]-ethyl}pyrrolidine-2-one or a pharmaceutically acceptable salt thereof as an active ingredient.

Yet another aspect of the present disclosure provides a method for treating diabetes including administering (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof in a pharmaceutically effective dose to a subject with diabetes.

Yet another aspect of the present disclosure provides a use of (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof, for use in the preparation of a pharmaceutical composition for preventing or treating diabetes.

Yet another aspect of the present disclosure provides a use of (R)-4-{(R)-1-[7-(3,4,5-trimethoxy-phenyl)-[1,6] naphthyridine-5-yloxy]-ethyl}pyrrolidine-2-one and cromolyn or pharmaceutically acceptable salts thereof, for use in the preparation of a pharmaceutical composition for preventing or treating diabetes.

### [Advantageous Effects]

According to the present disclosure, (R)-4-{(R)-1-[7-(3,4,5-trimethoxyphenyl)-[1,6] naphthyridine-5-yloxy]-ethyl}pyrrolidine-2-one of the present disclosure alleviates clinical symptoms of diabetes, reduces blood sugar, reduces blood glucose concentrations and insulin-resistance and reduces liver and kidney damages in an animal model of insulin-resistant diabetes, and thus can be effectively used for preventing or treating diabetes, especially insulin-resistant diabetes.

### [Description of Drawings]

FIG. 1 is a diagram confirming histopathological changes in liver, kidney, and pancreas of a normal control group and a negative control group.
FIG. 2 is a diagram confirming histopathological changes in liver and kidney according to a dose of (R)-4-{(R)-1-[7-(3,4,5-trimethoxy-phenyl)-[1,6] naphthyridine-5-yloxy] -ethyl }pyrrolidine-2-one (BI-1002494).

### [Best Mode for the Invention]

Hereinafter, an embodiment of the present disclosure will be described in detail with reference to the accompanying drawings. However, the following embodiments are presented as examples for the present disclosure, and when it is determined that a detailed description of well-known technologies or configurations known to those skilled in the art may unnecessarily obscure the gist of the present disclosure, the detailed description thereof may be omitted, and the present disclosure is not limited thereto. Various modifications and applications of the present disclosure are possible within the description of claims to be described below and the equivalent scope interpreted therefrom.

Terminologies used herein are terminologies used to properly express embodiments of the present disclosure, which may vary according to a user, an operator's intention, or customs in the art to which the present disclosure pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present disclosure, unless otherwise defined, have the meaning as commonly understood by those skilled in the related art of the present disclosure. In addition, although desired methods and samples are described herein, similar or equivalent methods and samples thereto are also included in the scope of the present disclosure. The contents of all publications disclosed as references in this specification are incorporated in the present disclosure.

In one aspect, the present disclosure relates to a pharmaceutical composition for preventing or treating diabetes containing (R)-4-{(R)-1-[7-(3,4,5-trimethoxyphenyl)-[1,6] naphthyridine-5-yloxy]-ethyl}pyrrolidine-2-one or a pharmaceutically acceptable salt thereof as an active ingredient.

In one embodiment, the (R)-4-{(R)-1-[7-(3,4,5-trimethoxy-phenyl)-[1,6] naphthyridine-5-yloxy]-ethyl}pyrrolidine-2-one may be a compound represented by Chemical Formula 1 below:

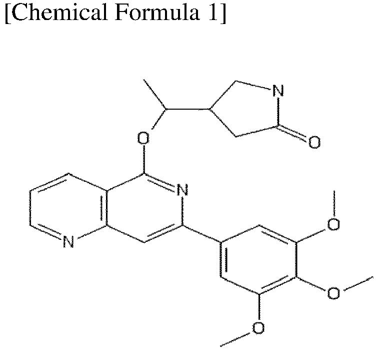

In one embodiment, the diabetes may be insulin-resistant diabetes, and may be Type 2 diabetes.

In one embodiment, the diabetes may be Type 1 diabetes, Type 2 diabetes, and gestational diabetes.

In one embodiment, the composition of the present disclosure may reduce blood sugar, insulin resistance, and liver and kidney damages.

In one embodiment, the composition of the present disclosure may further include cromolyn or cromolyn sodium (OC-101).

In one embodiment, the cromolyn may be a compound represented by Chemical Formula 2 below:

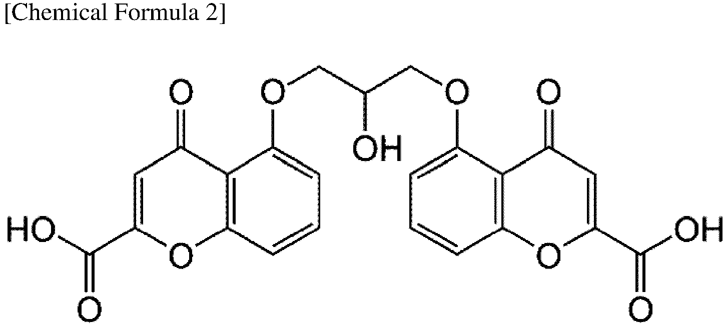

In one embodiment, a pharmaceutically acceptable salt of the cromolyn may be selected from the group consisting of alkali metal salts, alkaline earth metal salts, salts with inorganic acids, salts with organic acids, and salts with acidic amino acids, and most desirably cromolyn sodium.

In one embodiment, the (R)-4-{(R)-1-[7-(3,4,5-trimethoxy-phenyl)-[1,6] naphthyridine-5-yloxy]-ethyl}pyrrolidine-2-one and the cromolyn may be administered simultaneously, separately, or sequentially.

In one embodiment, the (R)-4-{(R)-1-[7-(3,4,5-trimethoxy-phenyl)-[1,6] naphthyridine-5-yloxy]-ethyl}pyrrolidine-2-one and the cromolyn may each be contained in the composition at a concentration of 5 to 100 mg/kg.

In one aspect, the present disclosure relates to a pharmaceutical composition for preventing or treating diabetic complications containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

In one embodiment, the diabetic complications may be one or more selected from the group consisting of diabetic retinopathy, diabetic stroke, diabetic cardiovascular disease, diabetic lung disease, diabetic peripheral neuropathy, diabetic wound healing delay, and diabetic cancer metastasis.

In an aspect, the present disclosure relates to a pharmaceutical composition for preventing or treating diabetes-related metabolic abnormalities containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

In one embodiment, the diabetes-related metabolic abnormalities may be selected from the group consisting of hyperglycemia, liver damage, kidney damage, pancreas damage, and blood lipid abnormalities.

The present disclosure includes not only a compound represented by Chemical Formula 1 or 2, but also pharmaceutically acceptable salts thereof, and all solvates, hydrates, racemates or stereoisomers, which are able to be prepared therefrom.

The (R)-4-{(R)-1-[7-(3,4,5-trimethoxy-phenyl)-[1,6] naphthyridine-5-yloxy]-ethyl}pyrrolidine-2-one represented by Chemical Formulas 1 and 2 of the present disclosure and the cromolyn may each be used in the form of pharmaceutically acceptable salts, and an acid addition salt formed by a pharmaceutically acceptable free acid is useful as the salt. The acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxy alkanoates and alkanes, dioates, aromatic acids, aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate.

The acid addition salts according to the present disclosure may be prepared by a conventional method, for example, by dissolving the compound in an excess acidic aqueous solution and precipitating the salt using a water-miscible organic solvent, such as methanol, ethanol, acetone or acetonitrile. In addition, the acid addition salts may also be prepared by evaporating and drying a solvent or excess acid from the mixture or by suction-filtering the precipitated salt. In addition, pharmaceutically acceptable metal salts may be prepared using bases. Alkali metal salts or alkaline earth metal salts may be obtained, for example, by dissolving the compound in an excess alkali metal hydroxide or alkaline earth metal hydroxide solution, filtering a non-dissolved compound salt, and then evaporating and drying a filtrate. At this time, as the metal salt, it is pharmaceutically suitable to prepare a sodium, potassium or calcium salt. Further, silver salts corresponding thereto may be obtained by reacting the alkali metal or alkaline earth metal salt with a suitable silver salt (e.g., silver nitrate).

The pharmaceutical composition of the present disclosure may further include known diabetes therapeutic agents, in addition to the (R)-4-{(R)-1-[7-(3,4,5-trimethoxy-phenyl)-[1,6] naphthyridine-5-yloxy]-ethyl}pyrrolidine-2-one and the cromolyn or the salts thereof as an active ingredient, and may be used in combination with other known treatments for the treatment of these diseases.

As used herein, the term "prevention" means all actions that inhibit or delay the occurrence, spread, and recurrence of metabolic diseases by administration of the pharmaceutical composition according to the present disclosure. The term "treatment" means all actions that improve or beneficially change the symptoms of metabolic diseases by administering the composition of the present disclosure. Those skilled in the art to which the present disclosure pertains will be able to determine the degree of alleviation, enhancement and treatment by knowing the exact criteria of a disease for which the composition of the present disclosure is effective by referring to data presented by the Korean Academy of Medical Sciences, etc.

As used herein, the term "therapeutically effective dose" used in combination with the active ingredients means an amount effective to prevent or treat a target disease, and the therapeutically effective dose of the composition of the present disclosure may vary depending on several factors, such as a method of administration, a target site, and the condition of a patient. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present disclosure is administered in a pharmaceutically effective dose. As used herein, the term "pharmaceutically effective dose" refers to an amount enough to treat diseases at a reasonable benefit/risk ratio applicable to medical treatment and enough to not cause side effects. The effective dose level may be determined according to factors including a health condition of a patient, a type of diabetes, an occurrence cause of diabetes, severity, drug activity, sensitivity to a drug, an administration method, an administration time, an administration route and excretion rate, a treatment period, and drugs used in combination or concurrently, and other factors well-known in medical fields. The composition of the present disclosure may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present disclosure may include carriers, diluents, excipients, or a combination of two or more thereof, which are commonly used in biological agents. As used herein, the term "pharmaceutically acceptable" means that the composition exhibits non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared desirably according to each disease or ingredient using as a suitable method in the art or a method disclosed in Remington's Pharmaceutical Science (Mack Publishing Company, Easton PA, 18th, 1990).

In one embodiment, the pharmaceutical composition may be one or more formulations selected from the group consisting of oral formulations, external preparations, suppositories, sterile injections and sprays, and more desirably oral or injective formulations.

As used herein, the term "administration" means providing a predetermined substance to a subject or patient by any suitable method, and parenteral administration (e.g., applied in an injection formulation intravenously, subcutaneously, intraperitoneally or topically) or oral administration may be performed according to a desired method. The dose range may vary depending on the body weight, age, sex, and health condition of a patient, a diet, an administration time, an administration method, an excretion rate, and the severity of a disease. Liquid formulations for oral administration of the composition of the present disclosure correspond to suspensions, internal solutions, emulsions, syrups, etc., and may include various excipients, such as wetting agents, sweeteners, fragrances, and preservatives in addition to water and liquid paraffin, which are commonly used simple diluents. Formulations for parenteral administration include sterilized aqueous solutions, non-aqueous solvents, suspensions, emulsions, lyophilized agents, and suppositories. The pharmaceutical composition of the present disclosure may also be administered by any device capable of transferring an active substance to a target cell. Desired methods of administration and formulations are intravenous injections, subcutaneous injections, intradermal injections, intramuscular injections, or drop injections. The injections may be prepared by using aqueous solvents such as a physiological saline solution and a ringer solution, and non-aqueous solvents such as vegetable oils, higher fatty acid esters (e.g., ethyl oleate), and alcohols (e.g., ethanol, benzyl alcohol, propylene glycol, or glycerin). The injections may include pharmaceutical carriers, such as a stabilizer for the suppression of degeneration (e.g., ascorbic acid, sodium hydrogen sulfite, sodium pyrosulfite, BHA, tocopherol, and EDTA), an emulsifier, a buffer for pH control, and a preservative to inhibit microbial growth (e.g., phenyl mercury nitrate, thimerosal, benzalkonium chloride, phenol, cresol, and benzyl alcohol).

As used herein, the term "subject" refers to all animals including monkeys, cows, horses, sheep, pigs, chickens, turkeys, quails, cats, dogs, mice, rats, rabbits or guinea pigs including humans who have developed or may develop the diabetes, and the pharmaceutical composition of the present disclosure may be administered to a subject to effectively prevent or treat the diseases. The pharmaceutical composition of the present disclosure may be administered in combination with existing therapeutic agents.

The pharmaceutical composition of the present disclosure may further include pharmaceutically acceptable additives. At this time, the pharmaceutically acceptable additives may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present disclosure is desirably included in an amount of 0.1 part by weight to 90 parts by weight based on the composition, but is not limited thereto.

In one aspect, the present disclosure relates to a food composition for preventing or alleviating diabetes containing (R)-4-{(R)-1-[7-(3,4,5-trimethoxyphenyl)-[1,6] naphthyridine-5-yloxy]-ethyl}pyrrolidine-2-one or a pharmaceutically acceptable salt thereof as an active ingredient.

In one embodiment, the (R)-4-{(R)-1-[7-(3,4,5-trimethoxy-phenyl)-[1,6] naphthyridine-5-yloxy]-ethyl}pyrrolidine-2-one may be a compound represented by Chemical Formula 1 below:

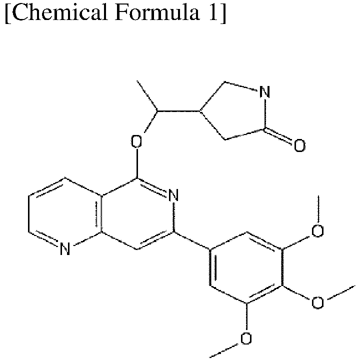

In one embodiment, the diabetes may be insulin-resistant diabetes, and may be Type 2 diabetes.

In one embodiment, the diabetes may be Type 1 diabetes, Type 2 diabetes, and gestational diabetes.

In one embodiment, the composition of the present disclosure may reduce blood sugar, insulin resistance, and liver and kidney damages.

In one embodiment, the composition of the present disclosure may further include cromolyn.

When the composition of the present disclosure is used as the food composition, the composition may be added as it is or used with other foods or food ingredients, and may be appropriately used according to a general method. The composition may include food acceptable supplement additives in addition to the active ingredients, and the mixing amount of the active ingredients may be appropriately determined depending on the purpose of use (prevention, health or therapeutic treatment).

As used herein, the term "food supplement additive" means a component that may be supplementally added to food, and may be appropriately selected and used by those skilled in the art as being added to prepare a health functional food of each formulation. Examples of the food supplement additives include various nutrients, vitamins, minerals (electrolytes), flavors such as synthetic and natural flavors, colorants and fillers, pectic acid and salts thereof, alginic acid and salts thereof, organic acids, protective colloidal thickeners, pH adjusters, stabilizers, preservatives, glycerin, alcohols, and carbonation agents used in carbonated drinks, but the types of food supplement additives of the present disclosure are not limited by the examples.

The food composition of the present disclosure may include a health functional food. As used herein, the term "health functional food" refers to food prepared and processed in the form of tablets, capsules, powders, granules, liquids and pills using raw materials or ingredients having functionalities useful to the human body. Here, the 'functionality' means regulating nutrients to the structure and function of the human body or obtaining effects useful for health applications such as physiological action. The health functional food of the present disclosure is able to be prepared by methods to be commonly used in the art and may be prepared by adding raw materials and ingredients which are commonly added in the art in preparation. In addition, the formulations of the health functional food may also be prepared with any formulation recognized as a health functional food without limitation. The food composition of the present disclosure may be prepared in various types of formulations, and unlike general drugs, the food composition has an advantage that there is no side effect that may occur when taking a long-term use of the drug using the food as a raw material, and has excellent portability, but the health functional food of the present disclosure may be taken as supplements to enhance the effects of fibrosis or cancer therapeutic agents.

In addition, there is no limitation in a type of health food in which the composition of the present disclosure may be used. In addition, the composition including the compound of the present disclosure as the active ingredient may be prepared by mixing known additives with other suitable auxiliary ingredients that may be contained in the health functional food according to the selection of those skilled in the art. Examples of foods to be added include meat, sausage, bread, chocolate, candy, snacks, confectionery, pizza, ramen, other noodles, gum, dairy products including ice cream, various soups, beverages, tea, drinks, alcoholic beverages, and vitamin complexes, and may be prepared to be added to extract, tea, jelly, and juice prepared by using the compound according to the present disclosure as a main ingredient.

In one aspect, the present disclosure relates to a method for treating diabetes including administering (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof in a pharmaceutically effective dose to a subject with diabetes.

In one embodiment, the method may further include administering cromolyn or a pharmaceutically acceptable salt thereof in a pharmaceutically effective dose to a subject with diabetes.

In one aspect, the present disclosure relates to a use of (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof, for use in the preparation of a pharmaceutical composition for preventing or treating diabetes.

In one aspect, the present disclosure relates to a use of (R)-4-{(R)-1-[7-(3,4,5-trimethoxy-phenyl)-[1,6] naphthyridine-5-yloxy]-ethyl}pyrrolidine-2-one and cromolyn or pharmaceutically acceptable salts thereof, for use in the preparation of a pharmaceutical composition for preventing or treating diabetes.

### [Modes for the Invention]

Hereinafter, the present disclosure will be described in more detail with reference to the following Examples. However, the following Examples are only intended to embody the contents of the present disclosure, and the present disclosure is not limited thereto.

### Example 1. Production of animal model of insulin-resistant diabetes and administration of substances

STZ (Sigma-aldrich, S0130, 40 mg/kg b.w., in 0.1 M citrate buffer pH 4.5) was intraperitoneally administered to 9-week-old male mice (C57BL/6) once/1 day for 5 days to perform a first disease induction. After 7 weeks of the first induction, a second induction was performed using the same method as the first induction. For 5 days of an STZ administration period for the first induction, 10% sucrose was administered as drinking water. In addition, after 14 days of the second administration, blood sugar was confirmed to confirm the induction rate, and the induced animals were separated into groups. Experimental substances (R)-4-{(R)-1-[7-(3,4,5-trimethoxy-phenyl)-[1,6] naphthyridine-5-yloxy]-ethyl}pyrrolidine-2-one (BI-1002494) and cromolyn (OC-101) were administered orally into the stomach at 10 mL/kg using an oral administration syringe (sonde) before 16:00 daily for 12 weeks at a frequency of 7 days/week at doses shown in Table 1 below, respectively, and dexamethasone was orally administered as a positive control substance.

**[Table 1]**

| Group | | Administering substance | Dose (mg/kg b.w.) | Dosage amount (mL/kg ) | Sex | Animal number |
|---|---|---|---|---|---|---|
| G1 | Normal control | Sterile distilled water | - | 10 | Male | 10 |
| G2 | Negative control | Disease-inducing + Sterile distilled water | - | 10 | Male | 10 |
| G3 | Positive control | Disease-inducing + Dexamethasone | 2 | 10 | Male | 10 |
| G4 | Test substance administered group | Disease-inducing + BI-1002494 | 12.5 | 10 | Male | 10 |
| G5 | | Disease-inducing + BI-1002494 | 25 | 10 | Male | 10 |
| G6 | | Disease-inducing + BI-1002494 | 50 | 10 | Male | 10 |
| G7 | | Disease-inducing + OC-101 | 50 | 10 | Male | 10 |

### Example 2. Confirmation of clinical symptoms

### 2-1. Confirmation of body weights

As a result of confirming the body weight of mice in each group of Example 1, the body weight of a negative control group was statistically significantly reduced after week 7 of administration compared to that of a normal control group, and no statistically significant difference was observed in a positive control group. In test substance administered groups, as compared to the negative control group, in a 12.5 mg/kg administered group of (R)-4-{(R)-1-[7-(3,4,5-trimethoxy-phenyl)-[1,6] naphthyridine-5-yloxy]-ethyl}pyrrolidine-2-one, after week 5 of administration, the body weight was statistically significantly increased, and in 25 mg/kg and 50 mg/kg administered groups, after week 8, the body weights were statistically significantly increased (Table 2).

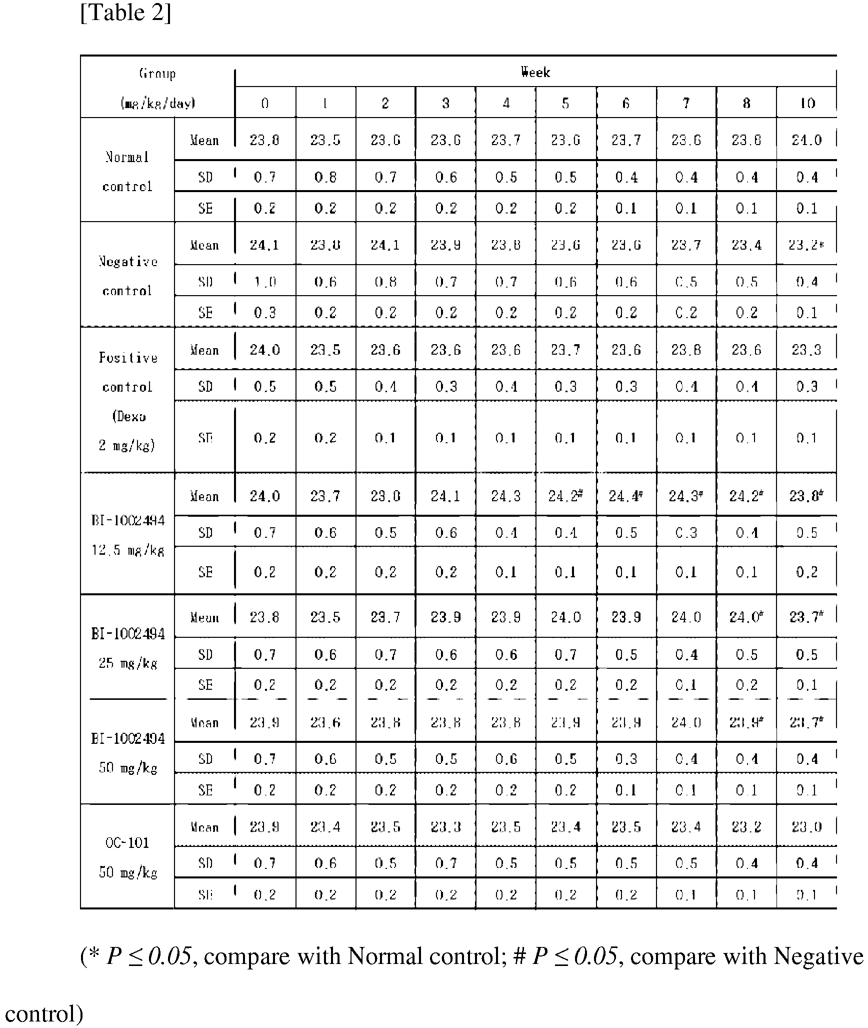

### 2-2. Confirmation of feed intake

As a result of confirming the feed intake of mice in each group of Example 1, the feed intake of a negative control group was statistically significantly increased after weeks 2, 3, 4, 6 and 8 of administration compared to that of a normal control group, and no statistically significant difference was observed in a positive control group compared to the normal control group. In test substance administered groups, as compared to the negative control group, in a 12.5 mg/kg administered group of (R)-4-{(R)-1-[7-(3,4,5-trimethoxy-phenyl)-[1,6] naphthyridine-5-yloxy]-ethyl}pyrrolidine-2-one, after week 4 of administration, in a 25 mg/kg administered group, after week 6 of administration, and in a 50 mg/kg administered group, after week 8 of administration, the feed intakes were statistically significantly decreased (Table 3).

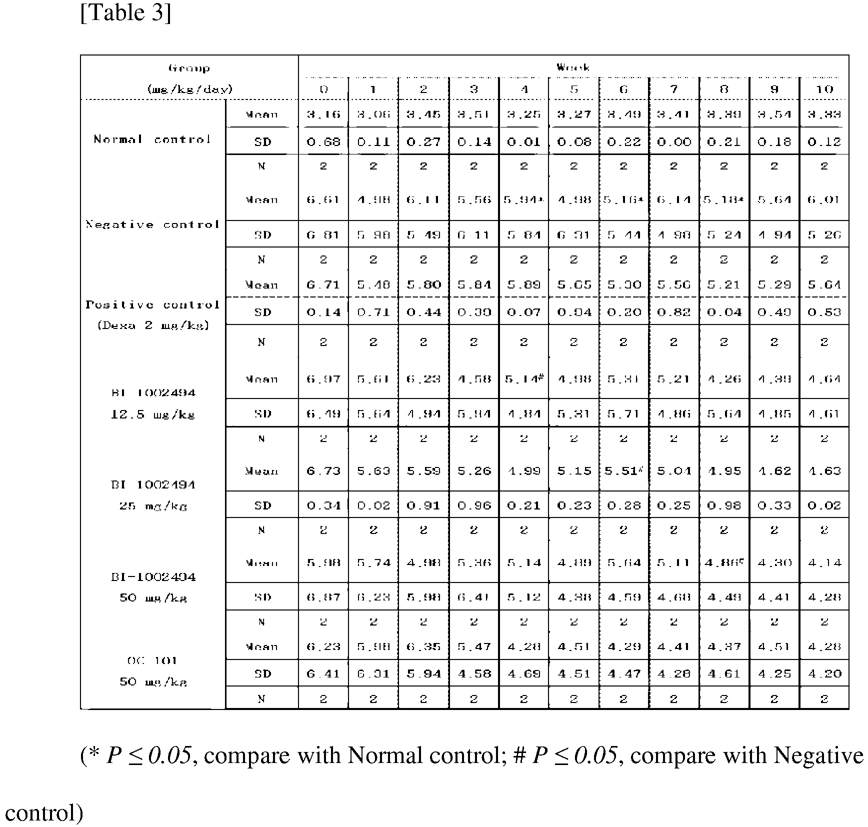

### 2-3. Confirmation of drinking water intake

As a result of confirming the drinking water intake of mice in each group of Example 1, the drinking water intake in the negative control group was statistically significantly increased at weeks 0 to 4, 6, and 7 of administration compared to that of the normal control group (Table 4).

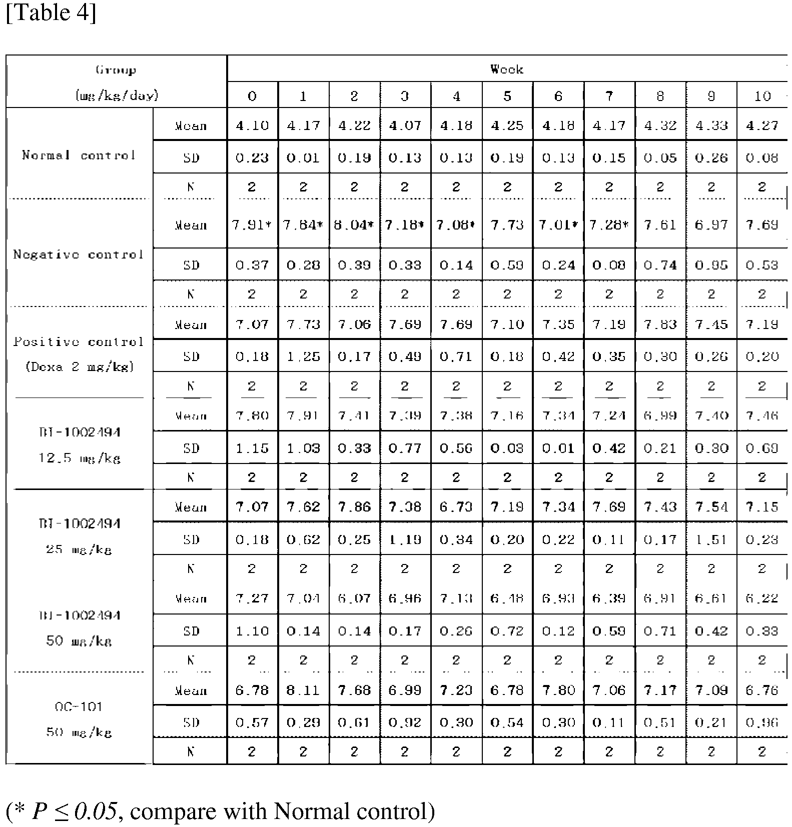

### Example 3. Confirmation of changes in blood sugar

Before disease induction, excluding week 9 of administration of experimental substances to mice in each group of Example 1 above, after 2 weeks after the first induction (based on a first administration date), and after 6 weeks and 2 weeks of the second induction (based on the first administration date), blood was collected from the tail vein and blood sugar was measured using a blood sugar meter (Accu-Check, Roche), and fasting was performed for 4 hours from 9:30 a.m. to 1:30 p.m.

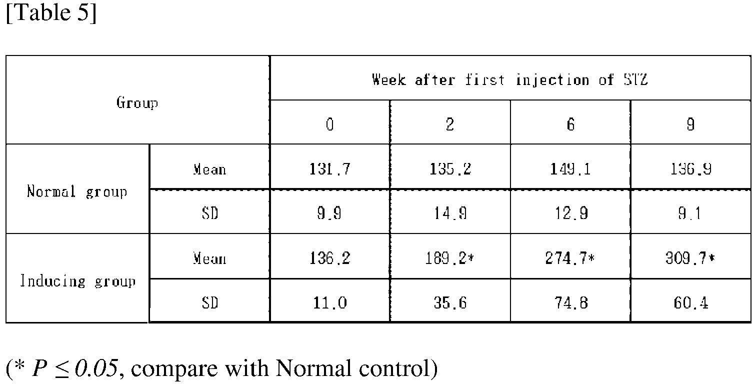

As a result, compared to the control group, a statistically significant increase in blood sugar was observed in a disease-inducing group from 2 weeks after the first STZ administration, and in such a change, a difference was increased even at week 6 and week 9 of administration (Table 5).

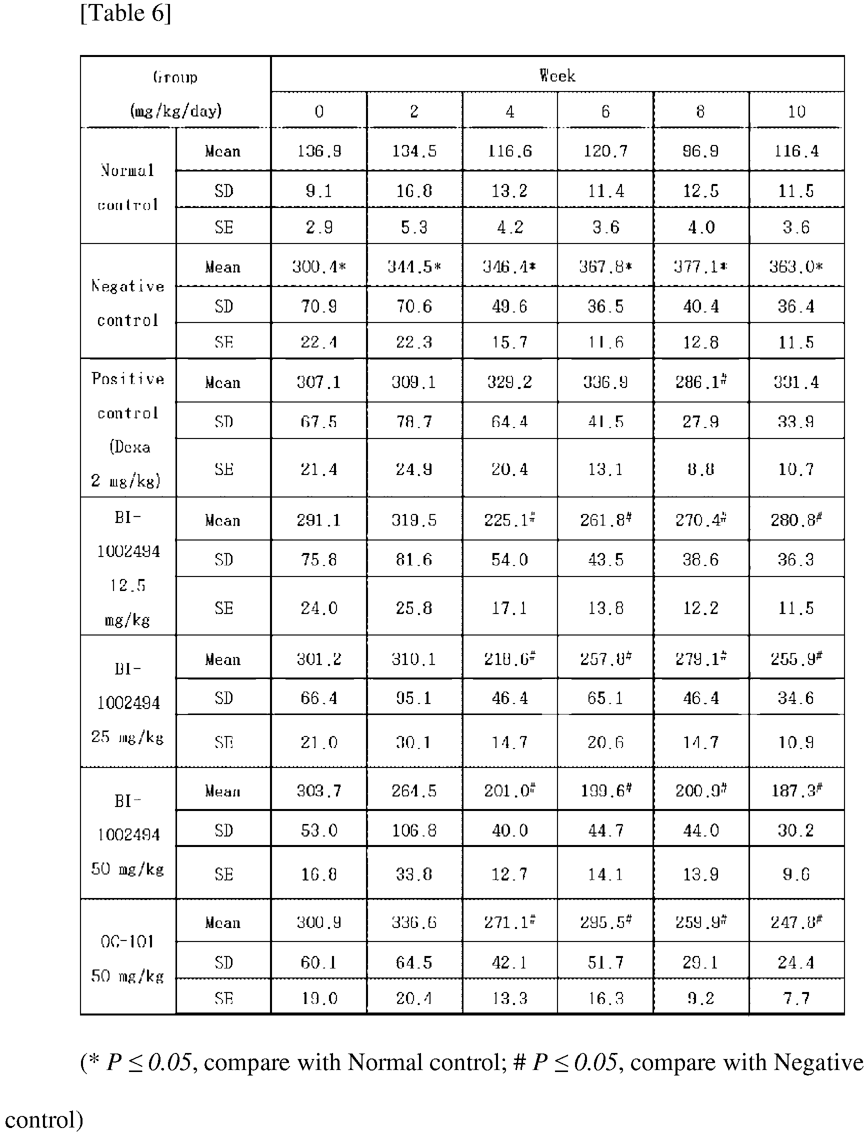

In addition, during the period before administration of the experimental substances, in the negative control group, blood sugar was increased statistically significantly compared to those of the normal control group, and in a positive control group, compared with the normal control group, after week 6 of administration, the blood sugar tended to be decreased, and at week 8 of administration, the blood sugar was decreased statistically significantly, and in the administered group of (R)-4-{ (R)-1-[7-(3,4,5-trimethoxy-phenyl)-[1,6] naphthyridine-5-yloxy]-ethyl}pyrrolidine-2-one, the blood sugar was decreased significantly compared to those of the positive control group (Table 6).

### Example 4. Confirmation of blood biochemical change

After the observation period for mice of each group in Example 1 was terminated, all surviving animals were fasted in the same manner as when measuring blood sugar, and then anesthetized by inhaling isoflurane, and blood was collected from the abdominal vena cava through laparotomy. After the blood collection was completed, the veins and arteries were cut under anesthesia and exsanguinated. The collected blood was left at room temperature for 30 minutes or more and then centrifuged at 3,000 rpm for 20 minutes. Thereafter, glucose, blood urea nitrogen (BUN), and creatinine were analyzed using an automated blood biochemistry analyzer (Hitachi 3100, Hitachi Co. Ltd., Japan). Indoxyl sulfate (a substance that was mainly produced in the intestines to cause kidney damage, and a substance that caused end-stage renal failure by advancing kidney function damage in patients with renal failure during hemodialysis) was analyzed using LC MS/MS. In addition, blood insulin was analyzed according to a method represented by a commercial Mouse Insulin ELISA KIT (#AKRIN-011T, Sibayagi Co. Ltd., Japan).

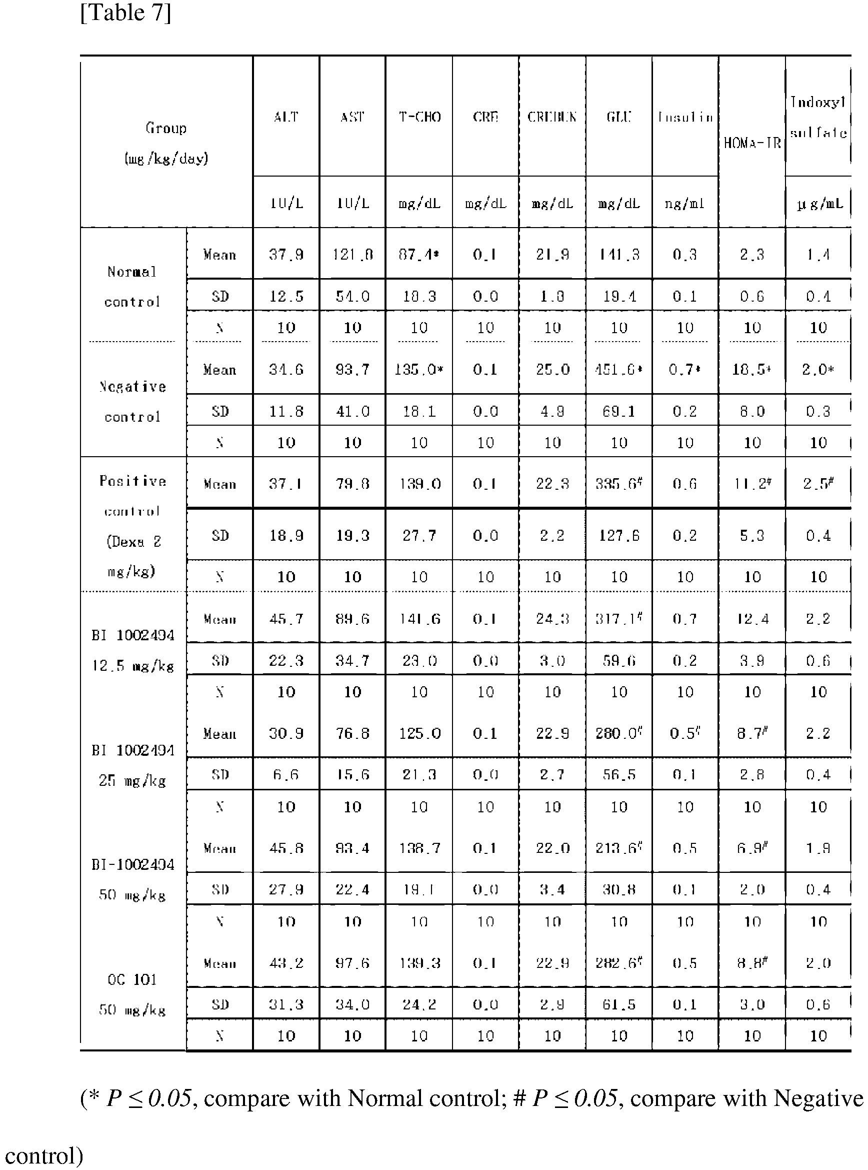

As a result, in the negative control group, T-CHO, GLU, insulin, HOMA-IR, and Indoxyl sulfate were increased statistically significantly compared to those of the normal control group, and in the positive control group, GLU and HOMA-IR were decreased statistically significantly and Indoxyl sulfate was increased statistically significantly compared to those of the negative control group (Table 7). In addition, in all administered groups of (R)-4-{(R)-1-[7-(3,4,5-trimethoxy-phenyl)-[1,6] naphthyridine-5-yloxy]-ethyl}pyrrolidine-2-one, GLU was statistically significantly reduced, and a clear dose dependence was observed. In addition, in 25 and 50 mg/kg administered groups of (R)-4-{(R)-1-[7-(3,4,5-trimethoxy-phenyl)-[1,6] naphthyridine-5-yloxy]-ethyl}pyrrolidine-2-one (BI-1002494), HOMA-IR was statistically significantly reduced as compared with that of the negative control group (Table 7). In addition, in the OC-101 administered group, GLU and HOMA-IR were decreased statistically significantly compared to those of the negative control group (Table 7).

### Example 5. Confirmation of histopathological change

At the end of the observation period, the mice in each group of Example 1 were confirmed to be dead, and the liver, kidney, and pancreas were extracted and fixed with 10% neutral buffered formalin (NBF) in at least 20 times the tissue volume. The fixed tissue was sectioned to a thickness of approximately 4 mm and then produced into a paraffin block through general tissue processing and embedding processes. Thereafter, the tissue was sectioned to a thickness of 4 µm using a rotary microtome, and then H&E staining was performed, and histopathological changes were observed.

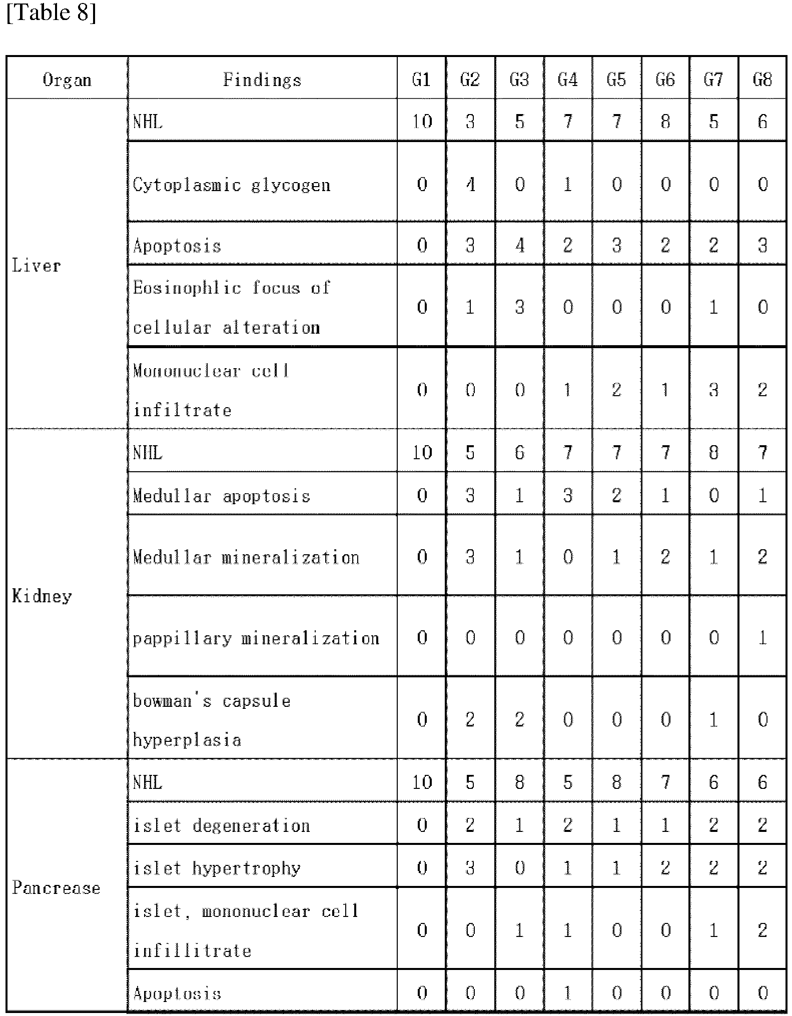

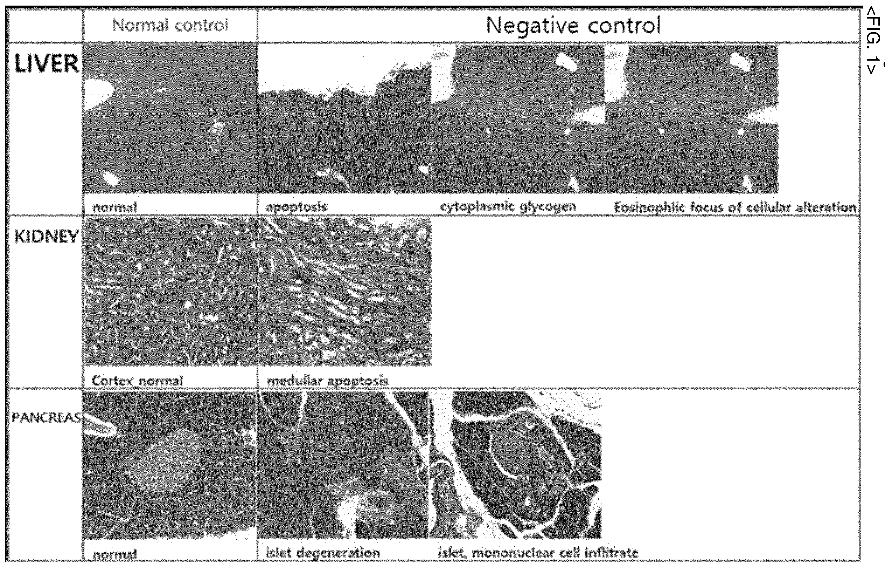

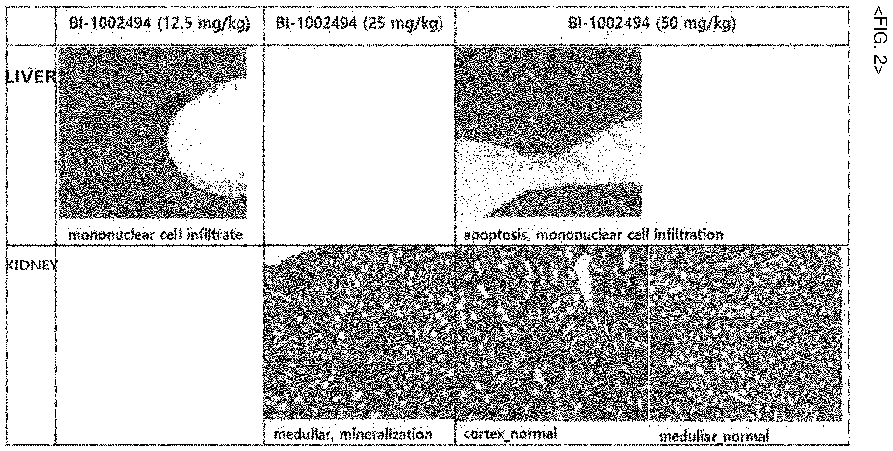

As a result, in the negative control group, compared to the normal control group, cytoplasmic glycogen, apoptosis, and the like were observed in the liver, medullar apoptosis and medullar mineralization were observed in the kidney, and islet degeneration and islet hypertrophy were observed in the pancreas (Table 8 and FIG. 1). Further, in the administered group of (R)-4-{(R)-1-[7-(3,4,5-trimethoxy-phenyl)-[1,6] naphthyridine-5-yloxy]-ethyl}pyrrolidine-2-one, the cytoplasmic glycogen was reduced in the liver and the medullar apoptosis was reduced in the kidney in a dose-dependent manner (Table 8 and FIG. 2).

Through the result, it was confirmed that in disease induction using STZ, in the negative control group, compared to the normal control group, blood glucose and insulin were increased, insulin resistance (HOMA-IP(IR)) was increased, the apoptosis of the kidney was confirmed, glomerular basement membrane hypertrophy was observed, and the features of diabetes were exhibited. In addition, when administering (R)-4-{(R)-1-[7-(3,4,5-trimethoxy-phenyl)-[1,6] naphthyridine-5-yloxy]-ethyl}pyrrolidine-2-one after such induction of diabetic diseases, it may be seen that blood sugar, and blood glucose and insulin resistance (HOMA-IR) were decreased in a dose-dependent manner, and (R)-4-{(R)-1-[7-(3,4,5-trimethoxyphenyl)-[1,6] naphthyridine-5-yloxy]-ethyl}pyrrolidine-2-one is effective in treatment of diabetic diseases.

## Claims

1. A pharmaceutical composition for preventing or treating diabetes comprising (R)-4- { (R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

2. The pharmaceutical composition for preventing or treating diabetes of claim 1, wherein the (R)-4-{(R)-1-[7-(3,4,5-trimethoxy-phenyl)-[1,6] naphthyridine-5-yloxy]-ethyl}pyrrolidine-2-one is a compound represented by Chemical Formula 1 below:

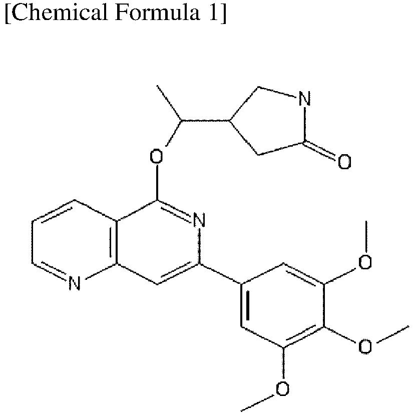

3. The pharmaceutical composition for preventing or treating diabetes of claim 1, wherein the diabetes is insulin-resistant diabetes.

4. The pharmaceutical composition for preventing or treating diabetes of claim 1, wherein the diabetes is Type 1 diabetes, Type 2 diabetes, and gestational diabetes.

5. The pharmaceutical composition for preventing or treating diabetes of claim 1, wherein the composition reduces blood sugar.

6. The pharmaceutical composition for preventing or treating diabetes of claim 1, wherein the composition reduces insulin resistance.

7. The pharmaceutical composition for preventing or treating diabetes of claim 1, wherein the composition reduces liver and kidney damages.

8. The pharmaceutical composition for preventing or treating diabetes of claim 1, further comprising cromolyn.

9. A pharmaceutical composition for preventing or treating diabetic complications containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

10. The pharmaceutical composition for preventing or treating diabetic complications of claim 9, wherein the diabetic complications are one or more selected from the group consisting of diabetic retinopathy, diabetic stroke, diabetic cardiovascular disease, diabetic lung disease, diabetic peripheral neuropathy, diabetic wound healing delay, and diabetic cancer metastasis.

11. A pharmaceutical composition for preventing or treating diabetes-related metabolic abnormalities containing (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

12. The pharmaceutical composition for preventing or treating diabetes-related metabolic abnormalities of claim 11, wherein the diabetes-related metabolic abnormalities are one or more selected from the group consisting of hyperglycemia, liver damage, kidney damage, pancreas damage, and blood lipid abnormalities.

13. A food composition for preventing or alleviating diabetes comprising (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof as an active ingredient.

14. The food composition for preventing or alleviating diabetes of claim 13, wherein the diabetes is insulin-resistant diabetes.

15. The food composition for preventing or alleviating diabetes of claim 13, further comprising cromolyn.

16. A method for treating diabetes comprising administering (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof in a pharmaceutically effective dose to a subject with diabetes.

17. The method for treating diabetes of claim 16, further comprising: administering cromolyn or a pharmaceutically acceptable salt thereof in a pharmaceutically effective dose to a subject with diabetes.

18. A use of (R)-4-{(R)-1-[7-(3,4,5-trimethoxy-phenyl)-[1,6] naphthyridine-5-yloxy]-ethyl}pyrrolidine-2-one or a pharmaceutically acceptable salt thereof, for use in the preparation of a pharmaceutical composition for preventing or treating diabetes.

19. A use of (R)-4-{(R)-1-[7-(3,4,5-trimethoxy-phenyl)-[1,6] naphthyridine-5-yloxy]-ethyl}pyrrolidine-2-one and cromolyn or pharmaceutically acceptable salts thereof, for use in the preparation of a pharmaceutical composition for preventing or treating diabetes.
